# EUROPEAN PATENT APPLICATION

(11) **EP 4 707 347 A1**
(43) Date of publication of application: **11.03.2026**
(21) Application number: 24811198.1
(22) Date of filing: 24.05.2024
(51) Int. Cl.: C09D 11/02, C07K 2/00

(54) **PRINTING INK, METHOD FOR PRODUCING PRINTED MATERIAL, AND PRINTED MATERIAL**

(30) Priority: 25.05.2023 JP 2023086311
(71) Applicant: Spiber Inc., Tsuruoka-shi, Yamagata 997-0052 (JP)
(72) Inventor: SEKIYAMA Kazuhide, Tsuruoka-shi, Yamagata 997-0052 (JP); TAKAMI Taku, Tsuruoka-shi, Yamagata 997-0052 (JP); SAKATA Kazuki, Tsuruoka-shi, Yamagata 997-0052 (JP)
(74) Representative: Handley, Matthew Edward
(86) International application number: PCT/JP2024/019212
(87) International publication number: WO 2024/242194

(57) **Abstract**

Disclosed is a printing ink containing: a fixing agent containing a peptide polymer having a polypeptide chain; a liquid medium; and a coloring agent. Also disclosed is a method for producing a printed material, the method including: forming a film containing a printing ink on a base material by a printing method; and removing a solvent from the film to form a print film containing a fixing agent and a coloring agent. The polypeptide chain may contain an artificial protein.

## Description

### Technical Field

The present disclosure relates to a printing ink, a method for producing a printed material, and a printed material.

### Background Art

A general method for producing a printed material includes forming a print film on various base materials such as paper by a printing method using a printing ink containing a coloring agent and a fixing agent. The fixing agent is a component that fixes the coloring agent to the base material, and often contains a synthetic resin (for example, Patent Literature 1).

### Citation List

### Patent Literature

Patent Literature 1: JP 2008-266494 A
Patent Literature 2: WO 2021/187502 A

### Summary of Invention

### Technical Problem

The present disclosure relates to a printing ink capable of forming a print film that can have biodegradability.

### Solution to Problem

The present disclosure includes the following:
[1] A printing ink containing: a fixing agent containing a peptide polymer having a polypeptide chain; a liquid medium; and a coloring agent.
[2] The printing ink according to [1], wherein the polypeptide chain contains an artificial protein.
[3] The printing ink according to [1] or [2], wherein the peptide polymer further has a modifying group bonded to the polypeptide chain.
[4] The printing ink according to any one of [1] to [3], wherein the peptide polymer is a block copolymer containing a first segment containing the polypeptide chain and a second segment having a plasticizing function for the polypeptide chain.
[5] The printing ink according to any one of [1] to [4], wherein the peptide polymer is a block copolymer containing a first segment containing the polypeptide chain and a second segment containing at least one selected from the group consisting of a polyether group, a polyester group, a polycarbonate group, a polyamide group, and a polyol group.
[6] The printing ink according to any one of [1] to [5], wherein at least one of the fixing agent and the coloring agent is dissolved in the liquid medium.
[7] The printing ink according to any one of [1] to [5], wherein at least one of the fixing agent and the coloring agent is dispersed in the liquid medium.
[8] A method for producing a printed material, the method including:
   forming a film containing the printing ink according to any one of [1] to [7] on a base material by a printing method; and
   removing the liquid medium from the film to form a print film containing the fixing agent and the coloring agent.
[9] The method according to [8], wherein the base material is a fiber assembly, paper, a plastic member, a metal member, a ceramic, a glass member, a wooden member, or food.
[10] A printed material containing: a base material; and a print film provided on the base material, wherein
   the print film contains a fixing agent containing a peptide polymer having a polypeptide chain, and a coloring agent.
[11] The printed material according to [10], wherein the base material is a fiber assembly, paper, a plastic member, a metal member, a ceramic, a glass member, a wooden member, or food.

### Advantageous Effects of Invention

A print film that can have biodegradability can be formed. When a printed material including a base material having biodegradability is produced, a decrease in biodegradability of the printed material can be suppressed.

### Brief Description of Drawings

Fig. 1 is a photograph of a printed material.
Fig. 2 is a photograph of a bent printed material.
Fig. 3 is a photograph of a bent printed material.
Fig. 4 is a photograph of a printed material after an iron test.

### Description of Embodiments

The present invention is not limited to the following examples.

An example of a printing ink contains a fixing agent containing a peptide polymer having a polypeptide chain, a liquid medium, and a coloring agent. The fixing agent may be dissolved or dispersed in the liquid medium. The printing ink can be used in order to form a print film on a base material by a printing method. That is, a printed material including the base material and the print film can be produced using the printing ink.

The peptide polymer constituting the fixing agent may be a polymer containing only a polypeptide chain which is an amino acid polymer, or may be a polymer further containing another molecular group (molecule) and/or a modifying group bonded to the polypeptide chain. The fixing agent may contain only the peptide polymer or may further contain other components (polymers).

The polypeptide chain may be a protein. The protein as the polypeptide chain may be a natural protein or an artificial protein. In the present specification, the natural protein means a protein having an amino acid sequence identical to an amino acid sequence of a naturally occurring protein, and the artificial protein means a protein having an amino acid sequence different from an amino acid sequence of a naturally occurring protein.

The polypeptide chain may be hydrophobic. The hydrophobic polypeptide chain can contribute to improvement of water resistance of a print film. The hydrophobicity of the polypeptide chain can be evaluated using a value of average hydropathy index (hydrophobic degree: hydrophobicity index) as an index. The average hydropathy index of the hydrophobic polypeptide chain may be, for example, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. The average hydropathy index of the hydrophobic polypeptide chain may be 1.00 or less, or 0.7 or less. The average hydropathy index of the hydrophobic polypeptide chain may be 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more, and 1.00 or less.

A value of average hydropathy index (HI) of the polypeptide chain or the protein and a value of hydropathy index of a repetitive sequence unit described later are determined according to a known method using a known hydrophobicity index of an amino acid residue. A known hydrophobicity index of an amino acid residue is presented in Table 1. For example, a value of hydrophobic degree (HI) can be calculated according to a method described in Kyte J & Doolittle R (1982) "A simple method for displaying the hydropathic character of a protein", J. Mol. Biol., 157, pp. 105 to 132.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The content of alanine residues in the polypeptide chain or the protein may be, for example, 10 to 40%, 12 to 40%, 15 to 40%, 18 to 40%, 20 to 40%, or 22 to 40% from a viewpoint of improving strength of a print film formed from the printing ink. The content of glycine residues in the polypeptide chain or the protein may be, for example, 10 to 55%, 11 to 55%, 13 to 55%, 15 to 55%, 18 to 55%, 20 to 55%, 22 to 55%, or 25 to 55%.

In the present specification, the "content of alanine residues" is a value represented by the following formula.

Content of alanine residues = (Number of alanine residues contained in polypeptide/Number of all amino acid residues of polypeptide) × 100(%)

The content of glycine residues, the content of serine residues, the content of threonine residues, the content of proline residues, and the content of tyrosine residues have the same meanings as those obtained by replacing the alanine residue with the glycine residue, the serine residue, the threonine residue, the proline residue, and the tyrosine residue, respectively, in the above formula.

The total content of proline residues, threonine residues, and tyrosine residues in any consecutive 20 amino acid residues in the polypeptide chain or the protein may be 5% or more, more than 5.5%, 6.0% or more, more than 6.5%, 7.0% or more, more than 7.5%, 8.0% or more, more than 8.5%, 9.0% or more, 10.0% or more, or 15.0% or more, and may be 50% or less, 40% or less, 30% or less, or 20% or less. The total content of proline residues, threonine residues, and tyrosine residues in any consecutive 20 amino acid residues in the polypeptide chain or the protein may be 5% or more, and 50% or less, 40% or less, 30% or less, or 20% or less.

In the polypeptide chain or the protein, the total content of serine residues, threonine residues, and tyrosine residues may be 4% or more, 4.5% or more, 5% or more, 5.5% or more, 6% or more, 6.5% or more, or 7% or more, and may be 35% or less, 33% or less, 30% or less, 25% or less, or 20% or less. The total content of serine residues, threonine residues, and tyrosine residues may be 4% or more, and 35% or less, 33% or less, 30% or less, 25% or less, or 20% or less.

The polypeptide chain or the protein may contain a plurality of amino acid sequences (repetitive sequence units) having high sequence identity with each other. The number of amino acid residues of the repetitive sequence unit may be 6 to 200. The sequence identity between the repetitive sequence units may be, for example, 85% or more, 90% or more, 95% or more, 96% or more, 97% or more, 98% or more, or 99% or more. A hydropathy index of the repetitive sequence unit may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. The hydropathy index of the repetitive sequence unit may be 1.0 or less, or 0.7 or less. The hydropathy index of the repetitive sequence unit may be -0.80 or more, -0.70 or more, -0.60 or more, - 0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more, and 1.0 or less.

The polypeptide chain or the protein may contain an (A)ₙ motif which is an amino acid sequence mainly containing alanine residues. The number of amino acid residues in the (A)ₙ motif may be an integer of 2 to 27, 2 to 20, 2 to 16, or 2 to 12. A ratio of the number of alanine residues to the number of all amino acid residues in the (A)ₙ motif may be 40% or more, 60% or more, 70% or more, 80% or more, 83% or more, 85% or more, 86% or more, 90% or more, 95% or more, or 100%.

The polypeptide chain or the protein may contain a glycine residue, a serine residue, or an alanine residue, and a cysteine residue located at a position adjacent thereto. The cysteine residue may be located at a position adjacent to the glycine residue. The cysteine residue having a mercapto group enables particularly easy bond formation between a first segment and a second segment in a block copolymer described later. The cysteine residue may be located between the glycine residue, the serine residue, or the alanine residue and the glycine residue, the serine residue, or the alanine residue, or may be located between the serine residue and the glycine residue.

In the polypeptide chain or the protein, the cysteine residue may be located at a position adjacent to a hydrophobic amino acid residue. The cysteine residue may be located between the hydrophobic amino acid residue and an amino acid residue other than the hydrophobic amino acid residue. The cysteine residue may be located between the hydrophobic amino acid residue and the glycine residue, the serine residue, or the alanine residue, or between the hydrophobic amino acid residue and the glycine residue. The hydrophobic amino acid residue may be one selected from the group consisting of an isoleucine residue, a valine residue, a leucine residue, a phenylalanine residue, a methionine residue, and an alanine residue.

A molecular weight of the polypeptide chain or the protein may be, for example, 200 to 1000000, 300 to 900,000, 400 to 800,000, 500 to 700,000, 600 to 600,000, 1000 to 600,000, 3000 to 600,000, 5000 to 600,000, 10,000 to 600,000, or 5000 to 100,000.

The molecular weight of the polypeptide chain or the protein may be, for example, 1000 or more, 2000 or more, 3000 or more, 4000 or more, 5000 or more, 6000 or more, 7000 or more, 8000 or more, 9000 or more, 10000 or more, 20000 or more, 30000 or more, 40000 or more, 50000 or more, 60000 or more, 70000 or more, 80000 or more, 90000 or more, or 100000 or more, and may be 400000 or less, less than 360000, 300000 or less, or 200000 or less.

In the present specification, the molecular weight of the polypeptide chain or the protein is a value measured by sodium dodecyl sulfate-polyacrylamide gel electrophoresis (SDS-PAGE). The electrophoresis is performed in the following procedure. First, 200 µL of 2 M lithium chloride DMSO (manufactured by FUJIFILM Wako Pure Chemical Corporation) is added to 2 mg of a powder sample, and the mixture is stirred while being heated at 80°C for 60 minutes and further at 95°C for ten minutes to dissolve the sample therein. Thereafter, the sample is diluted to 50 times with a 10 M urea solution, further diluted to 2 times with a sample buffer (manufactured by FUJIFILM Wako Pure Chemical Corporation), and heated at 95°C for five minutes to denaturalize the protein. Subsequently, a gel for SDS-PAGE (manufactured by Bio-lad) is attached to an electrophoresis device (manufactured by Bio-lad), the device is filled with an SDS buffer, and the electrophoresis device is connected to a power supply device (manufactured by Biocraft). 10 µL of the denatured sample is added to each well of the gel for SDS-PAGE, and a current of 30 mA/sheet is allowed to flow for 30 minutes. After completion of the electrophoresis, the gel for SDS-PAGE is taken out from the device, immersed in Oriole fluorescent gel stain (manufactured by Bio-lad), and shaken for one hour. Thereafter, the gel is placed on a UV sample tray (manufactured by Bio-lad), and a stained image is acquired with a Gel Doc EZ gel imaging device (manufactured by Bio-lad).

The number of amino acid residues constituting the polypeptide chain or the protein may be, for example, 50 or more, 100 or more, 150 or more, 200 or more, 250 or more, 300 or more, 350 or more, 400 or more, 450 or more, or 500 or more, and may be 5000 or less, 4500 or less, 4000 or less, 3500 or less, 3000 or less, 2500 or less, 2000 or less, 1500 or less, or 1000 or less. The number of amino acid residues constituting the polypeptide chain or the protein may be 50 or more, 100 or more, 150 or more, 200 or more, 250 or more, 300 or more, 350 or more, 400 or more, 450 or more, or 500 or more, and 5000 or less.

The protein constituting the peptide polymer may be a structural protein, and examples thereof include a natural protein selected from spider silk, silkworm silk, keratin, collagen, elastin, and resilin, and an artificial protein including an amino acid sequence derived therefrom. The protein may be particularly artificial fibroin or artificial spider silk fibroin.

The peptide polymer may be a modifying polypeptide chain having a polypeptide chain and a modifying group bonded to the polypeptide chain. The modifying group of the modifying polypeptide chain may be a reactive functional group. The reactive functional group may be an electrophilic functional group, a nucleophilic functional group, or a combination thereof. The modifying group can, for example, be bonded to the polypeptide chain via an ester bond. Examples of the modifying group that can be introduced into the polypeptide chain include an acyl group. The peptide polymer having the modifying polypeptide chain can contribute to improving durability (for example, water resistance or weather resistance) of a print film.

The peptide polymer may be a block copolymer containing a first segment containing the polypeptide chain and a second segment bonded to the first segment. A print film formed from a printing ink containing this block copolymer as a fixing agent tends to have high flexibility and stretchability. The print film having high flexibility and stretchability can contribute to suppression of cracking, peeling, and the like of a print film due to deformation such as expansion and contraction and bending of the base material. Examples of the block copolymer described in the present specification include those similar to those described in WO 2021/187502 A.

The block copolymer as the peptide polymer may contain a plurality of first segments and/or a plurality of second segments. The number of the second segments contained in the block copolymer may be 1 or 2 or more, 2 or more and 10 or less, 2 or more and 8 or less, 2 or more and 6 or less, or 2 or more and 4 or less with respect to one first segment.

The block copolymer may be a graft polymer having the first segment as a main chain and the second segment as a side chain. The first segment and the second segment may be alternately bonded. The block copolymer may contain a plurality of first segments, and one second segment may be bonded to two or more first segments to form a network structure.

The first segment and the second segment may be bonded to each other by, for example, a covalent bond, an ionic bond, a coordinate bond, or a combination thereof, or may be bonded to each other only by a covalent bond. A covalent bond may be formed between a linker group described later and the first segment.

The polypeptide chain contained in the first segment can be similar to the above-described examples of the polypeptide chain and the protein.

The second segment constituting the block copolymer (peptide polymer) may be a molecular group (molecule) having a plasticizing function for the polypeptide chain. The molecular group having a plasticizing function for the polypeptide chain means a molecular group having a function of enhancing flexibility of a molded body thereof as compared with a molded body containing only a polypeptide chain, such as a fiber or a film. A print film containing a block copolymer containing the second segment containing a molecular group having a plasticizing function for the polypeptide chain can have appropriate flexibility for a printed material. In addition, the block copolymer containing the second segment containing a molecular group having a plasticizing function for the polypeptide chain tends to have particularly favorable biodegradability.

The second segment constituting the block copolymer (peptide polymer) may be a molecular group containing at least one functional group selected from the group consisting of a polyether group, a polyester group, a polycarbonate group, a polyamide group, and a polyol group. A molecular chain containing these can have a plasticizing function for the polypeptide chain.

Examples of the polyether group include functional groups derived from polyalkylene glycols such as polyethylene glycol, polypropylene glycol, an ethylene oxide/propylene oxide copolymer, and polybutylene glycol. The functional group derived from polyalkylene glycols may be a polymer chain obtained by removing one or more hydrogen atoms from the polyalkylene glycols. When these polyether groups are contained in the second segment, the polyether group may be directly bonded to a hetero element (O, N, or S) in an ester group, a thioester group, or an amide group of a later-described linker contained in the second segment as necessary. Alternatively, the polyether group may be directly bonded to a hetero element (O, N, or S) in an ester group, a thioester group, or an amide group of a polypeptide skeleton contained in the first segment. In this case, the entire polyether group is easily separated from the linker or the first segment. As a result, a biodegradation rate of the polyether group can be increased.

Examples of the polyester group include functional groups derived from polyesters such as polylactic acid, poly(3-hydroxybutanoic acid), a polyhydroxybutanoic acid/hydroxyvaleric acid copolymer, a polyhydroxybutanoic acid/4-hydroxybutanoic acid copolymer, a polyhydroxybutanoic acid/hydroxyhexanoic acid copolymer, polytrimethylene terephthalate, a butanediol/long-chain dicarboxylic acid copolymer, polyethylene terephthalate, polybutylene succinate, a polybutylene succinate adipate copolymer, a polybutylene adipate terephthalate copolymer, polycaprolactone, and poly(trimethylene furandicarboxylate) (PTF). The functional group derived from the polyesters may be a polymer chain obtained by removing one or more hydrogen atoms from the polyesters. Among the polyesters described above, the polyester group may be a functional group derived from those classified into a biomass plastic such as polycaprolactone or a biodegradable plastic.

Examples of the polycarbonate group include functional groups derived from polycarbonates having an aliphatic hydrocarbon chain as a main skeleton, such as 1,6-hexanediol polycarbonate, 1,5-pentanediol polycarbonate, and 1,10 decanediol carbonate. The functional group derived from polycarbonates may be a polymer chain obtained by removing one or more hydrogen atoms from the polycarbonates.

Examples of the polyamide group include functional groups derived from polyamides such as nylon 3, nylon 4, nylon 5, nylon 6, nylon 11, and nylon 610 The functional group derived from polyamides may be a polymer chain obtained by removing one or more hydrogen atoms from the polyamides. Among the polyamides described above, the polyamide group may be a functional group derived from those classified into a biomass plastic or a biodegradable plastic.

Examples of the polyol group (polyvinyl alcohol group or the like) include functional groups derived from polyols (polyvinyl alcohols and the like) such as polyvinyl alcohol and an ethylene-vinyl alcohol copolymer. The residue derived from polyols may be a polymer chain obtained by removing one or more hydrogen atoms from the polyols. Among the polyols described above, the polyol group may be a functional group derived from those classified into a biomass plastic or a biodegradable plastic.

The second segment may further have a linker group interposed between the molecular group and the first segment. The linker group may be, for example, a divalent group represented by the following formula (1), (2a), (2b), (3a), (4a), (4b), (5), (6), (7), (8a), (8b), (9), (10), (11a), (11b), (13), (14), (15), or (16).

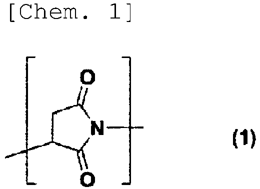

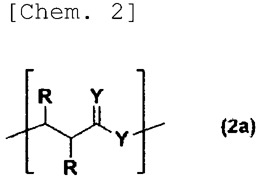

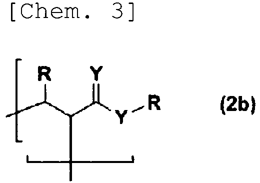

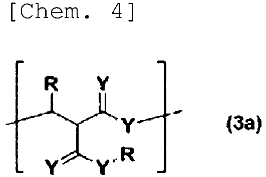

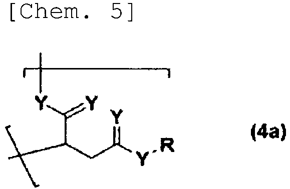

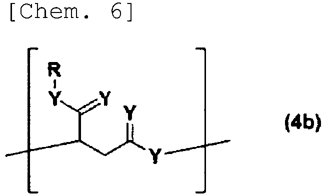

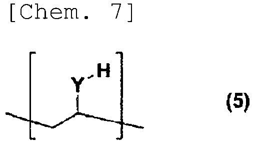

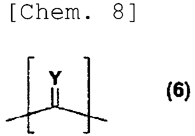

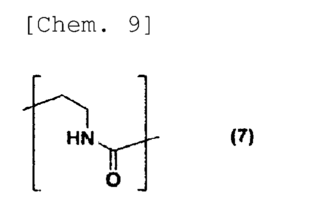

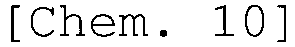

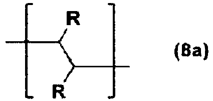

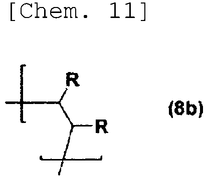

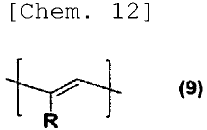

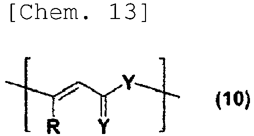

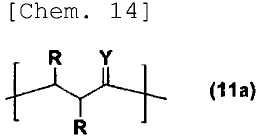

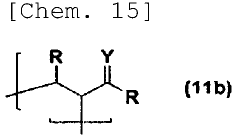

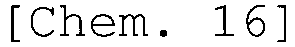

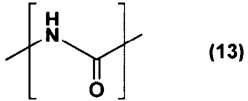

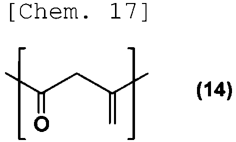

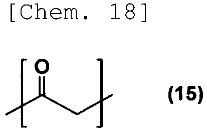

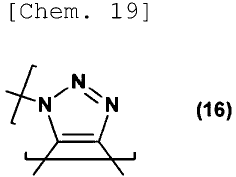

In the formulas (2a), (2b), (3a), (4a), (4b), (5), (6), (10), (11a), and (11b), Ys each independently represent an oxygen atom, a sulfur atom, or NR¹, and R¹ represents a hydrogen atom, a hydrocarbon group, an aromatic group, a carbonyl group, or a sulfonyl group. In the formulas (2a), (2b), (3a), (4a), (4b), (8a), (8b), (9), (10), (11a), and (11b), Rs each independently represent a hydrogen atom, a hydrocarbon group, or an aromatic group.

A molecular weight of the second segment may be 200 to 500,000, 300 to 400,000, 350 to 350,000, 400 to 300,000, 500 to 200,000, 600 to 1000000, 700 to 50,000, 800 to 10,000, 900 to 7500, or 1000 to 5000. The molecular weight herein may be a weight average molecular weight measured by GPC.

When a molecular weight of the polypeptide chain in the first segment is 100, a ratio of the molecular weight (or weight average molecular weight) of the second segment may be, for example, 1 to 10000, 1.5 to 9000, 2 to 8000, 3 to 7000, 5 to 5000, 7 to 3000, or 10 to 2000. A fact that the ratio of the molecular weight of the second segment to the molecular weight of the first segment is large can contribute to improvement of flexibility of a print film. A fact that the ratio of the molecular weight of the second segment to the molecular weight of the first segment is small can enhance rigidity of a print film.

In the block copolymer, a mass ratio of the content of the first segment when the content of the second segment is 100 may be, for example, 50 or more, 60 or more, 70 or more, 80 or more, 90 or more, 100 or more, 110 or more, 150 or more, 200 or more, 250 or more, 300 or more, 300 or more, 400 or more, 450 or more, 500 or more, 550 or more, or 600 or more, and may be 1000 or less, 900 or less, 800 or less, or 700 or less. The mass ratio of the content of the first segment when the content of the second segment is 100 may be, for example, 50 or more, 60 or more, 70 or more, 80 or more, 90 or more, 100 or more, 110 or more, 150 or more, 200 or more, 250 or more, 300 or more, 300 or more, 400 or more, 450 or more, 500 or more, 550 or more, or 600 or more, and 1000 or less.

The content of the fixing agent or the peptide polymer in the printing ink may be 4% by mass or more and 21% by mass or less on the basis of the total mass of the printing ink. The content of the fixing agent or the peptide polymer in the printing ink may be 7% by mass or more, 9% by mass or more, or 12% by mass or more, and may be 19% by mass or less, 16% by mass or less, or 14% by mass or less on the basis of the total mass of the printing ink.

The content of the fixing agent or the peptide polymer in the printing ink may be 12% by mass or more and 99% by mass or less on the basis of the total mass of components other than the liquid medium in the printing ink. The content of the fixing agent or the peptide polymer in the printing ink may be 25% by mass or more, 37% by mass or more, or 50% by mass or more, and may be 87% by mass or less, 74% by mass or less, or 62% by mass or less on the basis of the total mass of components other than the liquid medium in the printing ink. The content of the fixing agent or the peptide polymer in a print film formed from the printing ink can also be within these ranges.

The liquid medium contained in the printing ink can be selected from those that dissolve or disperse the peptide polymer favorably. The liquid medium may be, for example, an organic solvent in which the peptide polymer and/or the coloring agent can be dissolved, and examples thereof include dimethyl sulfoxide (DMSO), N,N-dimethylformamide (DMF), formic acid, an alcohol, and hexafluoroisopropanol (HFIP). The liquid medium may be, for example, water, a basic aqueous solution, an acidic aqueous solution, or an aqueous solution containing an inorganic salt or the like, in which the peptide polymer and/or the coloring agent can be dispersed.

The content of the liquid medium in the printing ink may be 37% by mass or more and 73% by mass or less on the basis of the total mass of the printing ink. The content of the liquid medium in the printing ink may be 42% by mass or more, 47% by mass or more, or 52% by mass or more, and may be 68% by mass or less, 63% by mass or less, or 57% by mass or less on the basis of the total mass of the printing ink.

The coloring agent contained in the printing ink may be a dye, a pigment, or a combination thereof.

The content of the coloring agent in the printing ink may be 0.1% by mass or more and 50% by mass or less on the basis of the total mass of the printing ink. The content of the coloring agent in the printing ink may be 1% by mass or more, 4% by mass or more, 7% by mass or more, 14% by mass or more, or 21% by mass or more, and may be 43% by mass or less, 36% by mass or less, or 29% by mass or less on the basis of the total mass of the printing ink.

The content of the coloring agent in the printing ink may be 0.7% by mass or more and 88% by mass or less on the basis of the total mass of components other than the liquid medium in the printing ink. The content of the coloring agent in the printing ink may be 3% by mass or more, 8% by mass or more, 13% by mass or more, 26% by mass or more, or 38% by mass or more, and may be 75% by mass or less, 63% by mass or less, or 50% by mass or less on the basis of the total mass of components other than the liquid medium in the printing ink. The content of the coloring agent in a print film formed from the printing ink can also be within these ranges.

If necessary, the printing ink may further contain other components in addition to the fixing agent, the liquid medium, and the coloring agent.

An example of a method for producing a printed material includes: forming a film containing the printing ink exemplified above on a base material by a printing method; and removing a liquid medium from the formed film to form a print film containing a fixing agent and a coloring agent. The printed material to be produced includes the base material and the print film provided on the base material. A certain amount of the liquid medium may remain in the print film.

A printing method that can be adopted is not particularly limited, but examples thereof include general plate printing such as relief printing, intaglio printing, plate printing, or stencil printing (for example, screen printing), plateless printing such as inkjet printing, and textile printing. Examples of the printing method also include printing in which a plate obtained by cutting out a design, a pattern, or the like to be printed is placed on a base material, and a printing ink is supplied thereon to form a print film having a design, a pattern, or the like similar to that cut out from the plate. The liquid medium may be removed, for example, by natural drying of a film containing the printing ink. For removal of the liquid medium, a film containing the printing ink may be heated. After a print film containing the printing ink according to the present disclosure is formed on a base material, on the print film, one or more print films containing at least one of the printing ink according to the present disclosure and a known printing ink may be formed. Alternatively, after a print film containing a known printing ink is formed on a base material, on the print film, one or more print films containing the printing ink according to the present disclosure may be formed.

The base material may be, for example, a fiber assembly, paper, leather, a plastic member, a metal member, a ceramic, a glass member, a wooden member, food, or a film. The fiber assembly may be, for example, a woven fabric, a knitted fabric, or a nonwoven fabric. When the base material is a fiber assembly containing a natural fiber, a regenerated fiber, a biodegradable synthetic fiber, an artificial protein fiber, or the like, paper, leather, a biodegradable plastic member, a wooden member, or food, the printed material itself tends to have appropriate biodegradability.

### Examples

The present invention is not limited to the following Examples.

### 1. Printing ink and formation of print film

### Example 1

Into a sample bottle containing a stirrer piece, 5.2 g (0.52 mmol) of an artificial protein (10 kDa) containing an (A)n motif which is an amino acid sequence mainly containing alanine residues and having an amino acid sequence in which the number of amino acid residues in the (A)n motif is 5 as a peptide polymer, 0.8 g of a pigment (Holbein GmbH, Carbon Black, Holbein PG341) as a coloring agent, 0.3 g of sodium dodecyl sulfate, and 24 g of formic acid as a liquid medium were put. The mixed liquid in the sample bottle was stirred with the stirrer piece at room temperature for 30 minutes. Thereafter, the mixed liquid was defoamed by centrifugation (manufactured by TOMY INDUSTRIAL CO., LTD., trade name: MX-307) to obtain a printing ink of Example 1 containing an artificial protein having a concentration of 17% by mass.

As a base material, each of a copy sheet (manufactured by ASKUL CORPORATION, a recycled copy paper R100, whiteness 80%) and a cotton fabric (Sojitz Fashion Co., Ltd., 182 cotton 40 smooth, 449 cloud cream) was attached to a stainless steel plate. On each of the base materials, 20 g of the printing ink was stretched with a doctor blade (gap 0.4 mm) to form a film of the printing ink on the base material. The base material on which the film of the printing ink was formed was allowed to stand in a draft chamber at room temperature for 12 hours to evaporate the liquid medium (formic acid) from the film of the printing ink. By evaporation of the liquid medium, a printed material having the print film fixed to the base material was formed.

### Example 2

As a peptide polymer, a block copolymer containing a first segment (artificial protein) which is a polypeptide chain containing an (A)n motif which is an amino acid sequence mainly containing alanine residues and having an amino acid sequence in which the number of amino acid residues in the (A)n motif is 5, and a second segment containing polyethylene glycol was prepared as follows for preparation.

To a two-necked eggplant flask, 6 g (0.6 mmol) of the artificial protein (10 kDa) as a peptide polymer and 85 mg (0.2 mmol) of dithiothreitol (DTT) as a reducing agent were added, and 63 g of dimethyl sulfoxide (DMSO) was added. These were heated and stirred at 85°C for 30 minutes to prepare a polypeptide solution. To the polypeptide solution, 6 g (0.6 mmol) of both terminal maleic acid esterified polyethylene glycol of 10 kDa was added, and the mixture was heated and stirred at 85°C for 30 minutes to obtain a dope solution containing a block copolymer.

Into a standard container having a capacity of 150 mL for a rotating and revolving mixer (THINKY CORPORATION, ARE-310), 6 g of dimethyl sulfoxide as a liquid medium, 1 g of a pigment (Holbein Art Materials Inc., carbon black, Holbein PG341) as a coloring agent, and 0.3 g of sodium dodecyl sulfate were put, and the mixed liquid in the standard container was treated in a mixing mode for five minutes. To the mixed liquid after the treatment, 40 g of the dope solution containing a block copolymer was added, and the mixed liquid was further treated in a mixing mode for five minutes using the same rotating and revolving mixer. Subsequently, the mixed liquid was treated in a defoaming mode for five minutes to obtain a printing ink of Example 2 containing a block copolymer containing an artificial protein and polyethylene glycol and having a concentration of 16% by mass.

As a base material, each of a copy sheet (manufactured by ASKUL CORPORATION, a recycled copy paper R100, whiteness 80%) and a cotton fabric (Sojitz Fashion Co., Ltd., 182 cotton 40 smooth, 449 cloud cream) was attached to a stainless steel plate. The whole cotton fabric was wetted with 10 g of water. Thereafter, on each of the base materials, 20 g of the printing ink was stretched with a doctor blade (gap 0.4 mm) to form a film of the printing ink on the base material. The base material on which the film of the printing ink was formed was heated at 60°C for six hours to evaporate the liquid medium (DMSO) from the film of the printing ink. By evaporation of the liquid medium, a printed material having the print film fixed to the base material was formed.

### Comparative Example 1

As a base material, each of a copy sheet (manufactured by ASKUL CORPORATION, a recycled copy paper R100, whiteness 80%) and a cotton fabric (Sojitz Fashion Co., Ltd., 182 cotton 40 smooth, 449 cloud cream) was attached to a stainless steel plate. On each of the base materials, 5 g of commercially available silkscreen printing ink (aqueous urethane rubber ink, Shin Nihon Zokei Co., Ltd., RUBADA, black) was stretched with a doctor blade (gap 0.4 mm) to form a film of the printing ink on the base material. The base material on which the film of the printing ink was formed was allowed to stand in a draft chamber at room temperature for 12 hours to evaporate the solvent (formic acid) from the film of the printing ink. By evaporation of the solvent, a printed material having the print film fixed to the base material was formed.

### 2. Appearance of printed material

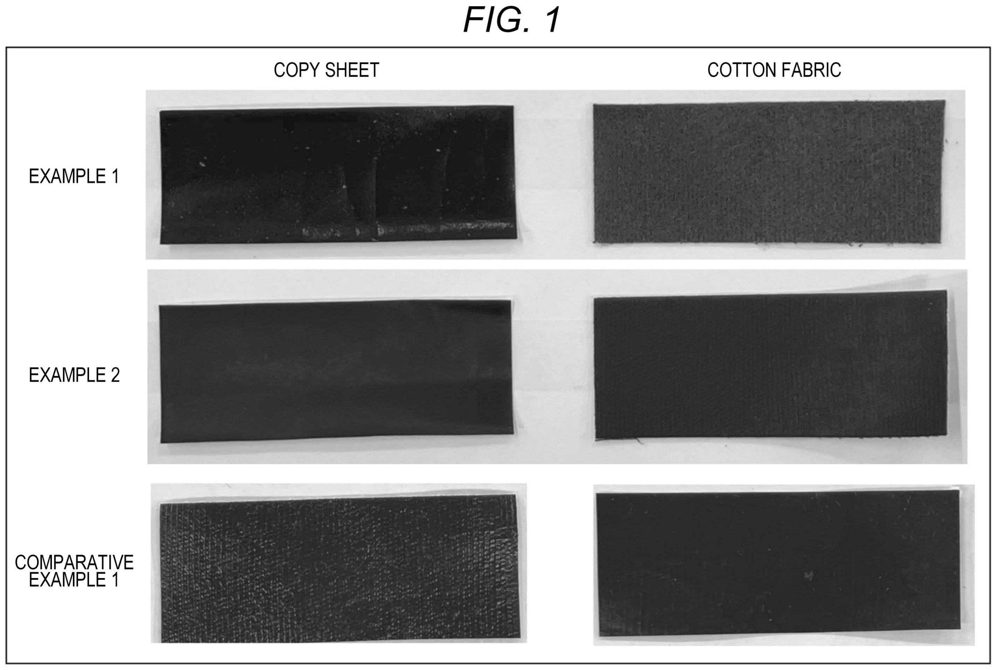

Fig. 1 is a photograph of a printed material obtained in Example or Comparative Example. Each of the printing inks of Examples 1 and 2 formed a uniform print film fixed to the base material in a similar manner to a commercially available printing ink. This indicates that a print film can be appropriately formed by forming a film of the printing ink in any region on the base material by various printing methods and drying the film.

### 3. Bending test

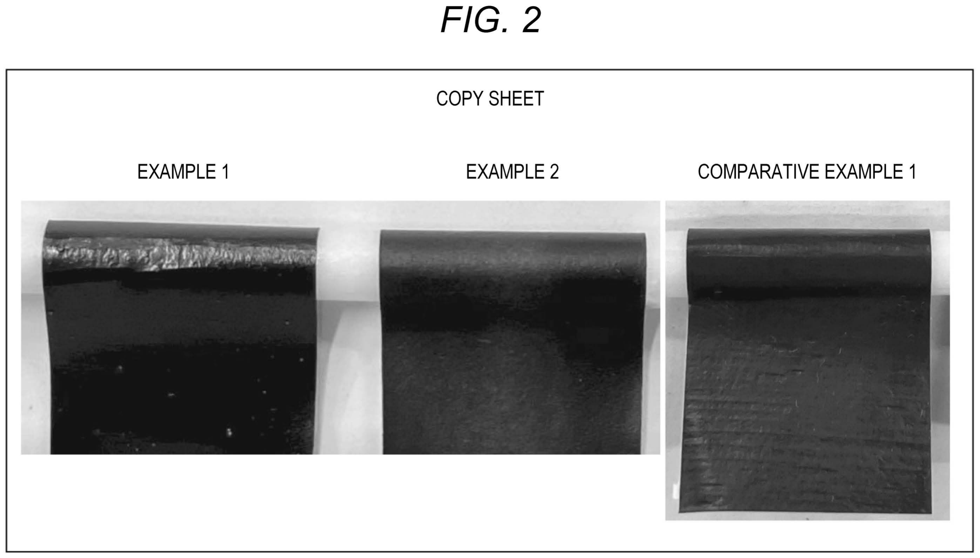

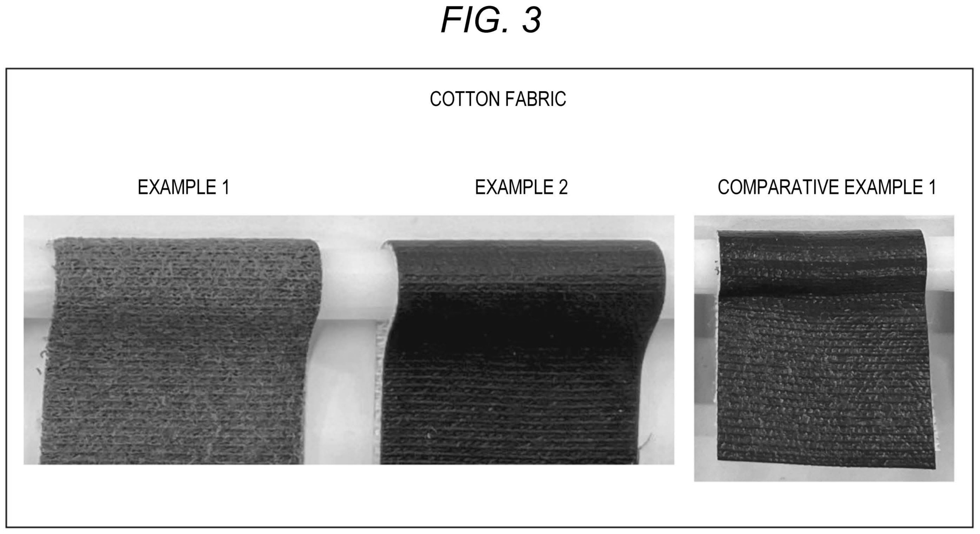

A test piece having a size of 2 cm × 5 cm was cut out from the obtained printed material including the base material and the print film obtained in each Example or Comparative Example. The test piece was bent so as to be wound around a polytetrafluoroethylene round bar (Flonchemical Co., Ltd.) having an outer diameter of 5 mm, and the print film was observed. Figs. 2 and 3 are photographs of bent printed materials. Fig. 2 illustrates a printed material having a copy sheet as a base material, and Fig. 3 illustrates a printed material having a cotton fabric as a base material. In any case, splits or cracks due to bending were not observed in the print film.

### 4. Iron test

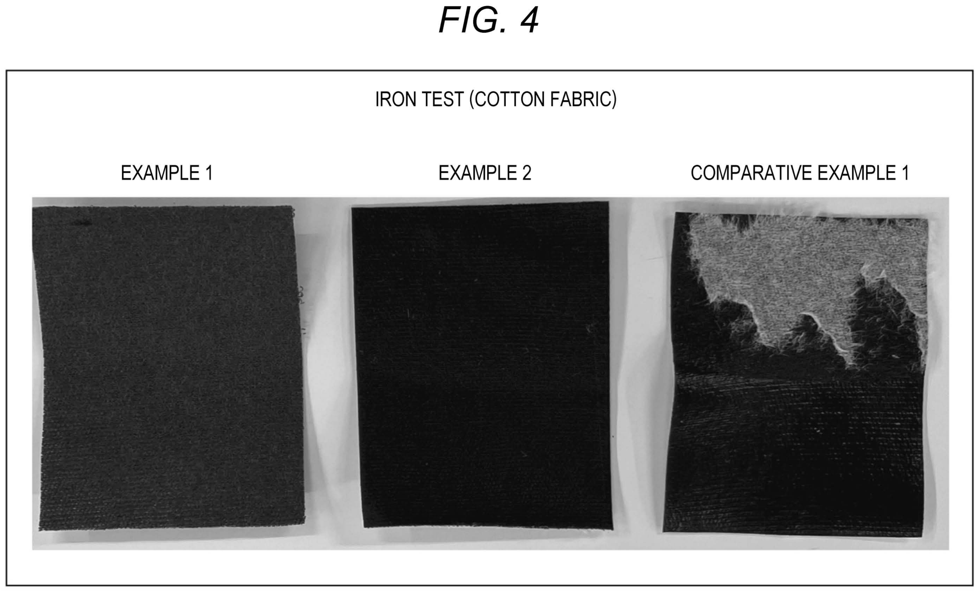

A 4 cm × 5 cm test piece for an iron test was cut out from a printed material having a cotton fabric, obtained in each Example or Comparative Example. A sheet of cloth (Elleair Soft Wiper S200, made of pulp) was placed on the print film of the test piece so as to cover a half region of the print film. A metal surface of an iron (Twinbird Corporation, SA-4084BL) heated to a high set temperature was pressed against a top of the cloth for ten seconds. Thereafter, the cloth was peeled off. Fig. 4 is a photograph of the printed material after the cloth was peeled off. In the case of Comparative Example 1, the print film was thermally melted by the iron, and thus the cloth that was not peeled off from the print film remained. In the case of Examples 1 and 2, the entire cloth could be peeled off without the cloth sticking to the print film. It was confirmed that the print film formed by the printing ink containing the peptide polymer had excellent heat resistance. For example, it is expected that a fabric decorated by the print film can be treated by an iron.

## Claims

1. A printing ink comprising: a fixing agent containing a peptide polymer having a polypeptide chain; a liquid medium; and a coloring agent.

2. The printing ink according to claim 1, wherein the polypeptide chain contains an artificial protein.

3. The printing ink according to claim 1, wherein the peptide polymer further has a modifying group bonded to the polypeptide chain.

4. The printing ink according to claim 1, wherein the peptide polymer is a block copolymer containing a first segment containing the polypeptide chain and a second segment having a plasticizing function for the polypeptide chain.

5. The printing ink according to claim 1, wherein the peptide polymer is a block copolymer containing a first segment containing the polypeptide chain and a second segment containing at least one selected from the group consisting of a polyether group, a polyester group, a polycarbonate group, a polyamide group, and a polyol group.

6. The printing ink according to claim 1, wherein at least one of the fixing agent and the coloring agent is dissolved in the liquid medium.

7. The printing ink according to claim 1, wherein at least one of the fixing agent and the coloring agent is dispersed in the liquid medium.

8. A method for producing a printed material, the method comprising:
forming a film containing the printing ink according to any one of claims 1 to 7 on a base material by a printing method; and
removing the liquid medium from the film to form a print film containing the fixing agent and the coloring agent.

9. The method according to claim 8, wherein the base material is a fiber assembly, paper, a plastic member, a metal member, a ceramic, a glass member, a wooden member, or food.

10. A printed material comprising: a base material; and a print film provided on the base material, wherein
the print film contains a fixing agent containing a peptide polymer having a polypeptide chain, and a coloring agent.

11. The printed material according to claim 10, wherein the base material is a fiber assembly, paper, a plastic member, a metal member, a ceramic, a glass member, a wooden member, or food.
